# EUROPÄISCHE PATENTSCHRIFT

(11) **EP 3 045 595 B1**
(45) Veröffentlichungstag und Bekanntmachung des Hinweises auf die Patenterteilung: **01.04.2020**
(21) Anmeldenummer: 15201505.3
(22) Anmeldetag: 21.12.2015
(51) Int. Cl.: E03C 1/10, F24D 17/00, G01N 33/18

(54) **VERFAHREN UND VORRICHTUNG ZUR DETEKTION VON STAGNIERENDEM WASSER**
METHOD AND DEVICE FOR THE DETECTION OF STAGNATING WATER
PROCÉDÉ ET DISPOSITIF DE DÉTÉCTION D'EAU STAGNANTE

(30) Priorität: 14.01.2015 DE 102015100457
(43) Veröffentlichungstag der Anmeldung: 20.07.2016
(73) Patentinhaber: Neoperl GmbH, 79379 Müllheim (DE)
(72) Erfinder: RUHNKE, Christof, 14959 Trebbin (DE)
(74) Vertreter: Rätsch, Caroline

(56) Entgegenhaltungen:
- EP-A1- 2 762 646
- DE-A1-102008 039 272
- DE-A1-102014 104 393

## Beschreibung

Die Erfindung betrifft ein Verfahren zur Detektion von stagnierendem Trinkwasser gemäß dem Oberbegriff des Patentanspruchs 1 und eine Vorrichtung zur Detektion von stagnierendem Trinkwasser gemäß dem Oberbegriff des Patentanspruchs 8, siehe z.B. DE102008039272A1 und DE102014104393A1.

Unter stagnierendem Wasser wird Wasser verstanden, das während eines bestimmten Zeitraums in einem Leitungsabschnitt steht. Stagnierendes Wasser tritt dann auf, wenn Wasser über einen bestimmten Zeitraum hinweg nicht entnommen (abgezapft) wird.

Bereits nach einigen Stunden kann es in stagnierendem Wasser aufgrund chemischer, physikalischer und mikrobieller Prozesse auf der Innenfläche des entsprechenden Leitungsabschnitts zur Bildung eines Biofilms kommen. Keime, die sich im Biofilm vermehren, gelangen bei einer Wasserentnahme an den Verbraucher. Hierdurch kann sich für den Verbraucher ein gesundheitliches Risiko ergeben.

Von der Verkeimung betroffen sind Leitungsabschnitte von z.B. Armaturen, Leitungen, Duschschläuchen und Hahnausläufen. Zur Vermeidung bzw. Reduzierung von Verkeimungen in Leitungsabschnitten sind aus dem Stand der Technik Ringleitungen bekannt, von denen relativ kurze Stichleitungen zu den Wasserentnahmestellen abgehen. Der Vorteil einer derartigen Konstruktion liegt darin, dass beim Abzapfen aus jeder Wasserentnahmestelle das Wasser in der Ringleitung bewegt wird, so dass die Verweildauer des Wassers in der Ringleitung gering ist und stagnierendes Wasser lediglich in den relativ kurzen Stichleitungen auftritt. Letzteres wird durch einen Spülvorgang ausgelassen, bevor das Trinkwasser (aus der Ringleitung) verwendet werden kann. Ringleitungen sind aufwendig und nicht überall realisierbar.

Auch materialtechnisch können Vorkehrungen getroffen werden, die eine Keimbildung reduzieren. Insbesondere sind aus dem Stand der Technik Materialien bekannt, die die Gefahr der Bildung eines Biofilms reduzieren.

Grundsätzlich kann die Gefahr der Benutzung von verkeimtem Trinkwasser dadurch reduziert werden, dass vor dem Abzapfen von zur Verwendung vorgesehenem Trinkwasser das Wasser einige Zeit laufen gelassen wird. Hierdurch wird die Leitung gespült und mögliches Stagnations-Wasser abgeführt. Je nach Länge der Wasserzuführleitung beträgt die Laufzeit des Wassers bis zu einigen Minuten.

Das vorbeschriebene Verfahren hat sich in der Praxis bewährt. Allerdings ist ein Ableiten von unbenutztem Wasser über einen längeren Zeitraum umweltunfreundlich, und zwar insbesondere dann, wenn aus Sicherheitsgründen das Wasser länger abgeführt wird als es eigentlich erforderlich wäre.

Auch zeigt die Praxis, dass vor der Benutzung einer Trinkwasser-Zapfstelle häufig nicht klar ist, ob es sich bei dem anstehenden Wasser um stagnierendes Wasser handelt oder nicht. Häufig sichert sich der Verbraucher dadurch ab, dass - unabhängig von dem Stagnationsgrad des Wassers - das Wasser grundsätzlich über längeren Zeitraum laufen gelassen wird, bevor es für seine eigentliche Verwendung abgezapft wird. Unnötige Wasserverschwendung ist die Folge.

Hier greift die Erfindung ein.

Der Erfindung liegt die **Aufgabe** zugrunde, stagnierendes Wasser zu detektieren bei gleichzeitiger Reduzierung von unnötigem Wasserverbrauch.

Die Aufgabe wird durch ein Verfahren mit den Merkmalen des Anspruchs 1 gelöst.

Die Erfindung vereint mehrere Vorteile. Ein Vorteil besteht darin, dass durch fließendes Wasser elektrische Energie erzeugt wird. Es ist also keine gesonderte Energiequelle erforderlich. Ein weiterer wesentlicher Vorteil der Erfindung besteht darin, dass aus dem Ladezustand des Energiespeichers eine Information darüber gewonnen werden kann, ob es sich um stagnierendes Wasser handelt oder nicht. Insbesondere nutzt die Erfindung einen entladenen Energiespeicher, um hieraus die Information zu gewinnen, dass es sich bei dem Wasser in dem Leitungsabschnitt um stagnierendes Wasser handelt.

Den Energiespeicher wird man vorzugsweise so wählen, dass seine Entladungszeit kürzer ist als die Zeit, in der das Wasser subjektiv oder objektiv als Stagnationswasser betrachtet wird. Beispielsweise beträgt die Entladungszeit weniger als 12 Stunden, vorzugsweise weniger als 6 Stunden. Je nach Einsatzzweck wird Trinkwasser bereits dann als Stagnationswasser definiert, wenn Wasser auf dem Weg im jeweiligen Leitungsabschnitt länger als 4 Stunden stillsteht. Derartige Bedingungen finden sich insbesondere in medizinischen Einrichtungen wie Krankenhäusern. Vor diesem Hintergrund kann es zweckmäßig sein, dass die Entladungszeit weniger als 4 Stunden beträgt.

Die Erfindung kann wie vorstehend angedeutet in öffentlichen Einrichtungen wie Krankenhäusern, Alten- und Pflegeheimen oder Schulen, aber auch in privaten Haushalten zum Einsatz kommen.

Vorzugsweise wird aus der Stagnation des Trinkwassers dessen Frischegrad abgeleitet. Es ist vorzugsweise also möglich, dass einerseits sichergestellt werden kann, dass das abgezapfte Wasser frisch ist, und dass andererseits nicht unnötiges Wasser (durch unnötiges Spülen der Leitung) verschwendet wird.

An dieser Stelle sei darauf hingewiesen, dass im Rahmen der Erfindung unter dem Frischegrad beispielsweise lediglich die Zustände "frisch" oder "nicht frisch" verstanden werden. In Weiterbildung der Erfindung können aber auch abgestufte Frischegrade ermittelt werden. Diese werden vorzugsweise aus einem teilweise entladenen Energiespeicher abgeleitet. Wenn also der Energiespeicher eine Restladung aufweist, kann hieraus abgeleitet werden, dass das abgezapfte Wasser "noch frisch" ist.

Vorteilhafterweise wird der Frischegrad des Wassers akustisch und/oder visuell angegeben. Der Frischegrad ist also für den Benutzer schnell erfassbar. Beispielsweise kann "frisches" Wasser mit einem Licht angezeigt werden, das z.B. grün oder blau ist. Zusätzlich oder alternativ kann ein Signalton oder eine Reihe von Signaltönen erfolgen. Bei "nicht frischem", also stagnierendem Wasser erfolgt vorzugsweise keine akustische und/oder visuelle Anzeige.

Alternativ und ebenfalls als vorteilhaft angesehen wird es, wenn Stagnationswasser visualisiert wird. Beispielsweise kann es sich um ein rotes Signal handeln. Das Wasser wird also dadurch als "frisch" angegeben, dass kein Signal erfolgt. Bei dieser Variante handelt es sich bei dem Signal also um ein Warnsignal für Stagnationswasser.

Sobald das Wasser über einen bestimmten, vorzugsweise einen vorgegebenen, Zeitraum in dem Leitungsabschnitt still steht, entlädt sich der Energiespeicher. Wird die Leitung bzw. der Leitungsabschnitt daraufhin gespült, lädt sich der Energiespeicher wieder auf. Vorteilhafterweise wird das Trinkwasser bei entladenem Energiespeicher nach einer bestimmten, vorzugsweise vorgegebenen, Auslaufzeit als "frisch" angegeben. In diesem Zusammenhang sind mehrere Szenarien denkbar. Beispielsweise wird das Wasser dann als "frisch" angegeben, wenn der Energiespeicher voll aufgeladen ist oder einen vorgegebenen Ladezustand erreicht hat. Auch möglich ist die Vorgabe einer konkreten Auslaufzeit, beispielsweise 1 Minute.

Als bevorzugt wird es angesehen, wenn die Auslaufzeit einstellbar ist. Hierdurch ist das Verfahren an seinen konkreten Einsatzort anpassbar. Sofern es beispielsweise bekannt ist, dass der Leitungsabschnitt zu der Wasserentnahmestelle lang ist, wird man die Auslaufzeit eher lang wählen. Bei einem kurzen Leitungsabschnitt, beispielsweise einer kurzen Stichleitung, kann die Auslaufzeit relativ kurz gehalten werden. Durch die Einstellbarkeit kann die Menge an Spülwasser, also an auslaufendem Stagnationswasser, möglichst gering gehalten werden. Eine derartige Wassereinsparung ist umweltfreundlich.

In der Praxis kann vorgesehen sein, dass die Auslaufzeit stufenlos einstellbar ist. Alternativ ist die Auslaufzeit in vorgegebenen Intervallen einstellbar.

Die vorbeschriebene Einstellbarkeit bietet eine einfache Möglichkeit, das erfindungsgemäße Verfahren an Umgebungsbedingungen anzupassen. Dabei wird eine gewisse Ortskenntnis vorausgesetzt. Eine genauere Einstellbarkeit kann dadurch geschaffen werden, dass zur Bestimmung der Auslaufzeit einmalig Wasser auslaufen gelassen wird, dass die Wassertemperatur während des Auslaufens gemessen wird und dass die Auslaufzeit auf Grundlage der Wassertemperaturänderung festgelegt wird. Der Hintergrund dieser Einstellmöglichkeit liegt darin, dass sich die Wassertemperatur bei Stillstand an die Umgebungstemperatur anpasst. Hierbei handelt es sich beispielsweise um die Raumtemperatur, durch die die jeweilige Leitung geht. In nord- und westeuropäischen Ländern ist die Raumtemperatur höher als die Temperatur des aus der zentralen Wasserversorgung stammenden "frischen" Wassers. Andererseits sind auch Länder in warmen Regionen bekannt, in denen aufgrund er Klimatisierung die Raumtemperatur geringer sein kann. Sobald eine bestimmte Temperaturänderung stattfindet, kann davon ausgegangen werden, dass Frischwasser an der Entnahmestelle ansteht. In der Praxis kann die Temperaturmessing beispielsweise manuell, also durch Fühlen der Temperatur, erfolgen. Sobald sich die gefühlte Wassertemperatur subjektiv ändert, ist hierdurch das Zeitintervall festgelegt, nach dem das Wasser "frisch" ist. Die so ermittelte Auslaufzeit kann für zukünftige Wasser-Entnahmevorgänge verwendet werden.

Als Energiespeicher kommen solche Speicher in Betracht, die elektrische Energie speichern und sich in einem geeigneten Zeitraum entladen. Beispielsweise kann ein Akkumulator zum Einsatz kommen. Da die Entladezeiten bei Akkumulatoren konstruktionsbedingt lang sind, können zusätzliche elektrische Bauteile (wie zum Beispiel eine optische Anzeige) vorgesehen sein, die vom Akkumulator gespeist werden. Hierdurch verkürzt sich die Entladezeit.

Als besonders vorteilhaft wird es angesehen, wenn der Energiespeicher als Kondensator ausgebildet ist. Derartige Kondensatoren können konstruktiv sehr klein gehalten werden. Darüber hinaus weisen Kondensatoren geeignete Lade- und Entladekurven auf.

Die Aufgabe wird ferner durch eine Vorrichtung mit den Merkmalen des Anspruchs 8 gelöst.

Der Generator wird vorzugsweise durch eine Turbine angetrieben. Die Turbine ist vorteilhaft Bestandteil der Vorrichtung. Sie ist zweckmäßig im Leitungsabschnitt angeordnet.

Die Steuer- und Auswerteinheit erfasst den Ladezustand des Energiespeichers. Der Signalgeber gibt in Abhängigkeit vom Ladezustand des Energiespeichers ein Signal ab. Wenn der Energiespeicher leer ist, gibt der Signalgeber kein Signal ab. Wenn der Energiespeicher voll ist, gibt der Signalgeber vorzugsweise ein Signal ab, insbesondere ein solches, dass das Wasser als "frisch" ausweist.

Vorteilhafterweise handelt es sich bei dem Signal um ein optisches Signal. Das Signal kann als permanentes oder blinkendes Lichtsignal ausgebildet sein. Beispielsweise ist es denkbar, dass "frisches" Wasser durch ein erstes, beispielsweise permanentes, Lichtsignal angezeigt wird. Je nach Entladezeit des Energiespeichers wird stagnierendes Wasser mit einem zweitem, beispielsweise andersfarbigem, Lichtsignal angezeigt. Wie bereits vorstehend erläutert, erfolgt bei entladenem Energiespeicher kein Signal, wobei zusätzlich vorteilhaft vorgesehen sein kann, dass während der Aufladung des Energiespeichers, während also noch stagnierendes Wasser abläuft, ein entsprechendes Signal abgegeben wird, das anders ein kann als das erste Signal, das für "frisches" Wasser steht.

Ebenfalls als vorteilhaft wird es angesehen, wenn nicht "frisches" Wasser signalisiert wird, sondern Stagnationswasser. Im Normalbetrieb, wenn also "frisches" Wasser an der Entnahmestelle anliegt, erfolgt vorteilhaft kein Signal. Wenn jedoch der Energiespeicher leer ist und stagnierendes Wasser entnommen wird, wird die elektrische Energie des Generators dazu genutzt, ein Signal auszugeben, dass es sich um Stagnationswasser handelt. Beispielsweise ist das Signal rot.

Die erfindungsgemäße Vorrichtung ist einfach und aus wenigen Teilen aufgebaut. Als besonders vorteilhaft wird es angesehen, wenn die Vorrichtung als End- oder Zwischenstück für einen Wasserauslauf, insbesondere für eine Wasserarmatur, ausgebildet ist. Die erfindungsgemäße Vorrichtung kann also auch als Nachrüstteil eingebaut werden. Ein weitergehender Umbau einer Standardarmatur ist hierzu nicht erforderlich. In diesem Zusammenhang vorteilhaft es auch, wenn der Energiespeicher als Kondensator ausgebildet ist, der eine besonders kleine Baugröße der erfindungsgemäßen Vorrichtung und eine Unterbringung in dem End- oder Zwischenstück gestattet.

In Weiterbildung der Erfindung wird vorgeschlagen, dass eine Eingabeeinrichtung vorgesehen ist, mittels der die Steuer- und Auswerteeinheit mit einem Zeitintervall gespeist werden kann. Bei dem Zeitintervall kann es sich um diejenige Zeit handeln, die das stagnierende Wasser benötigt, aus einem Leitungsabschnitt auszulaufen, bis es frisch ist. Bei entladenem Energiespeicher wird das Wasser - nach entsprechender Eingabe in die Steuer- und Auswerteinheit - erst nach dem Zeitintervall als "frisch" indiziert. Wie bereits zuvor erläutert, kann die Information über "frisches" Wasser durch ein aktives (z.B. optisches oder akustisches) Signal oder dadurch zur Verfügung gestellt werden, dass kein Signal abgegeben wird. In letzterem Fall liegt dann ein Signal an, wenn es sich um Stagnationswasser handelt.

Zusätzlich kann ein Temperaturfühler vorgesehen sein, der eine zusätzliche Überwachung des Wassers zur Verfügung stellt. Beispielsweise kann das auslaufende Stagnationswasser dahingehend überwacht werden, ob es - ggf. nach einem voreingestellten Zeitintervall - nach einer Temperaturänderung zu einer konstanten Wassertemperatur kommt.

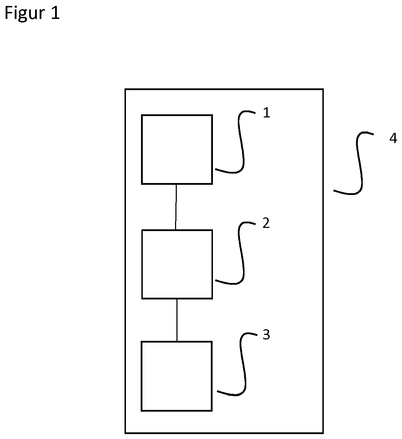

Im Folgenden wird die Erfindung anhand der anhängenden **Zeichnung** im Zusammenhang mit dem dargestellten Ausführungsbeispiel näher erläutert. Die Zeichnung zeigt in
- Figur 1:: in schematischer Darstellung den grundsätzlichen Aufbau einer erfindungsgemäßen Detektionsvorrichtung.

Die erfindungsgemäße Vorrichtung weist einen Generator 1 auf, der in einem (hier nicht dargestellten) Leitungsabschnitt angeordnet ist. Der Generator 1 wird über eine im durchströmten Leitungsabschnitt befindliche Turbine angetrieben und erzeugt hierdurch elektrische Energie. Die Energie wird in einem Energiespeicher 2 gespeichert, der beispielsweise als Kondensator ausgebildet sein kann. Mit dem Bezugszeichen 3 ist ein Signalgeber gekennzeichnet, der ein zum Beispiel optisches Signal abgibt, wenn an der Zapfstelle frisches Wasser anliegt. Die Bauteile 1, 2 und 3 können in einer Einheit 4 untergebracht sein, die beispielsweise ein End- oder Zwischenstück 4 für einen Wasserauslauf ist.

## Patentansprüche

1. Verfahren zur Detektion von stagnierendem Trinkwasser,
- wobei in einem Leitungsabschnitt durch fließendes Wasser elektrische Energie erzeugt wird,
**dadurch gekennzeichnet,**
- **dass** die elektrische Energie in einem Energiespeicher gespeichert wird,
- **dass** der Ladezustand des Energiespeichers erfasst wird, und
- **dass** aus dem Ladezustand des Energiespeichers Informationen über eine mögliche Stagnation des Trinkwassers gewonnen werden.

2. Verfahren nach Anspruch 1, **dadurch gekennzeichnet, dass** aus der Stagnation des Trinkwassers der Frischegrad abgeleitet wird.

3. Verfahren nach Anspruch 2, **dadurch gekennzeichnet, dass** der Frischegrad des Wassers vorzugsweise akustisch und/oder visuell angegeben wird.

4. Verfahren nach Anspruch 2 oder 3, **dadurch gekennzeichnet, dass** bei entladenem Energiespeicher das Trinkwasser nach einer vorgegebenen Auslaufzeit als "frisch" angegeben wird.

5. Verfahren nach Anspruch 4, **dadurch gekennzeichnet, dass** die Auslaufzeit einstellbar ist.

6. Verfahren nach Anspruch 4 oder 5, **dadurch gekennzeichnet, dass** zur Bestimmung der Auslaufzeit einmalig Wasser auslaufen gelassen wird, dass die Wassertemperatur während des Auslaufens gemessen wird und dass die Auslaufzeit auf Grundlage der Wassertemperaturänderung festgelegt wird.

7. Verfahren nach einem der Ansprüche 1 bis 6, **dadurch gekennzeichnet, dass** der Energiespeicher als Kondensator ausgebildet ist.

8. Vorrichtung zur Detektion von stagnierendem Trinkwasser, mit
- einem Generator (1) zur Erzeugung elektrischer Energie, **gekennzeichnet durch**
- einen Energiespeicher (2) zum Speichern der elektrischen Energie,
- eine Steuer- und Auswerteeinheit, die den Ladezustand des Energiespeichers erfasst, und
- einen Signalgeber (3), der in Abhängigkeit vom Ladezustand des Energiespeichers ein Signal abgibt.

9. Vorrichtung nach Anspruch 8, **dadurch gekennzeichnet, dass** es sich bei dem Signal um ein optisches Signal handelt.

10. Vorrichtung nach Anspruch 8 oder 9, **dadurch**
**gekennzeichnet, dass** sie als End- oder Zwischenstück (4) für einen Wasserauslauf, insbesondere eine Wasserarmatur, ausgebildet ist.

11. Vorrichtung nach einem der Ansprüche 8 bis 10, **dadurch gekennzeichnet, dass** der Energiespeicher (2) als Kondensator ausgebildet ist.

12. Vorrichtung nach einem der Ansprüche 8 bis 11,
**gekennzeichnet durch** eine Eingabeeinrichtung, mittels der die Steuer- und Auswerteeinheit mit einem Zeitintervall gespeist werden kann.

13. Vorrichtung nach einem der Ansprüche 8 bis 12,
**gekennzeichnet durch** einen zusätzlichen Temperaturfühler.

## Claims

1. Method for detecting stagnating potable water,
- wherein electrical energy is generated by flowing water in a line section,
**characterized**
- **in that** the electrical energy is stored in an energy store,
- **in that** the state of charge of the energy store is detected, and
- **in that** information about possible stagnation of the potable water is obtained from the state of charge of the energy store.

2. Method according to Claim 1, **characterized in that** the degree of freshness is derived from the stagnation of the potable water.

3. Method according to Claim 2, **characterized in that** the degree of freshness of the water is preferably acoustically and/or visually indicated.

4. Method according to Claim 2 or 3, **characterized in that**, when the energy store is discharged, the potable water is indicated as "fresh" after a prespecified drain time.

5. Method according to Claim 4, **characterized in that** the drain time can be adjusted.

6. Method according to Claim 4 or 5, **characterized in that** water is allowed to drain once for the purpose of determining the drain time, **in that** the water temperature during draining is measured, and **in that** the drain time is defined on the basis of the change in water temperature.

7. Method according to one of Claims 1 to 6, **characterized in that** the energy store is in the form of a capacitor.

8. Apparatus for detecting stagnating potable water, comprising
- a generator (1) for generating electrical energy, **characterized by**
- an energy store (2) for storing electrical energy,
- a control and evaluation unit which detects the state of charge of the energy store, and
- a signal transmitter (3) which outputs a signal depending on the state of charge of the energy store.

9. Apparatus according to Claim 8, **characterized in that** the signal is an optical signal.

10. Apparatus according to Claim 8 or 9, **characterized in that** it is in the form of an end or intermediate piece (4) for a water outlet, in particular a water fitting.

11. Apparatus according to one of Claims 8 to 10, **characterized in that** the energy store (2) is in the form of a capacitor.

12. Apparatus according to one of Claims 8 to 11, **characterized by** an input device by means of which the control and evaluation unit can be fed with a time interval.

13. Apparatus according to one of Claims 8 to 12, **characterized by** an additional temperature sensor.

## Revendications

1. Procédé de détection d'eau potable stagnante,
- de l'énergie électrique étant générée par écoulement d'eau dans une portion de conduite, **caractérisé en ce que**
- l'énergie électrique est stockée dans un acumulateur d'énergie,
- l'état de charge de l'accumulateur d'énergie est détecté, et
- des informations sur une éventuelle stagnation de l'eau potable sont obtenues à partir de l'état de charge de l'accumulateur d'énergie.

2. Procédé selon la revendication 1, **caractérisé en ce que** le degré de fraîcheur est dérivé de la stagnation de l'eau potable.

3. Procédé selon la revendication 2, **caractérisé en ce que** le degré de fraîcheur de l'eau est de préférence indiqué par moyens acoustiques et/ou visuels.

4. Procédé selon la revendication 2 ou 3, **caractérisé en ce que**, lorsque l'accumulateur d'énergie est déchargé, l'eau potable est indiquée comme « fraîche » après un temps d'écoulement spécifié.

5. Procédé selon la revendication 4, **caractérisé en ce que** le temps d'écoulement est réglable.

6. Procédé selon la revendication 4 ou 5, **caractérisé en ce que**, pour déterminer le temps d'écoulement, on laisse l'eau s'écouler une seule fois, **en ce que** la température de l'eau est mesurée pendant l'écoulement et **en ce que** le temps d'écoulement est déterminé en fonction de la variation de la température de l'eau.

7. Procédé selon l'une des revendications 1 à 6, **caractérisé en ce que** l'accumulateur d'énergie est conçu comme un condensateur.

8. Dispositif de détection d'eau potable stagnante, ledit dispositif comprenant
- un générateur (1) destiné à générer de l'énergie électrique, **caractérisé par**
- un accumulateur d'énergie (2) destiné à stocker l'énergie électrique,
- une unité de commande et d'évaluation qui détecte l'état de charge de l'accumulateur d'énergie, et
- un générateur de signaux (3) qui délivre un signal en fonction de l'état de charge de l'accumulateur d'énergie.

9. Dispositif selon la revendication 8, **caractérisé en ce que** le signal est un signal optique.

10. Dispositif selon la revendication 8 ou 9, **caractérisé en ce que** celui-ci est conçu comme une pièce d'extrémité ou intermédiaire (4) destinée à une sortie d'eau, notamment un raccord d'eau.

11. Dispositif selon l'une des revendications 8 à 10, **caractérisé en ce que** l'accumulateur d'énergie (2) est conçu comme un condensateur.

12. Dispositif selon l'une des revendications 8 à 11, **caractérisé par** un moyen d'entrée permettant d'alimenter l'unité de commande et d'évaluation avec un intervalle de temps.

13. Dispositif selon l'une des revendications 8 à 12, **caractérisé par** un capteur de température supplémentaire.
